# EUROPEAN PATENT APPLICATION

(11) **EP 4 494 550 A1**
(43) Date of publication of application: **22.01.2025**
(21) Application number: 23771092.6
(22) Date of filing: 15.03.2023
(51) Int. Cl.: A61B 5/00, A61B 5/103, G16H 50/20

(54) **METHOD FOR DETERMINING COLOR OF OBJECT, DEVICE THEREFOR, AND RECORDING MEDIUM STORING COMMANDS THEREFOR**

(30) Priority: 15.03.2022 KR 20220031821
(71) Applicant: Medit Corp., Seoul 07207 (KR)
(72) Inventor: BAEK, Jong Woong, Seoul 07207 (KR); CHO, Young Mok, Seoul 07207 (KR)
(74) Representative: MFG Patentanwälte Meyer-Wildhagen Meggle-Freund Gerhard PartG mbB
(86) International application number: PCT/KR2023/003473
(87) International publication number: WO 2023/177213

(57) **Abstract**

There is provided a method performed by an electronic device, the method including generating a three-dimensional image of an object based on a two-dimensional image set of the object obtained from an intraoral scanner, the object including a plurality of unit objects, based on a user's selection for a first point of the three-dimensional image, extracting a first color information set corresponding to a first region including the first point, using an artificial intelligence model configured to output a color similarity based on an input of the first color information set, and determining representative color information of a first unit object corresponding to the first point based on a magnitude of the color similarity.

## Description

### TECHNICAL FIELD

The present disclosure relates to technology for determining the color of an object (e.g., a patient's teeth).

### BACKGROUND

When producing dental prostheses (e.g., inlays, crowns, implants, etc.), it is necessary to determine the color of the dental prostheses so that they blend naturally with the patient's teeth.

In a conventional method for determining the color of the dental prostheses, the dentist visually observed and selected a guide of the most similar color to the patient's teeth from a plurality of predetermined color guides (e.g., Vita Classic Shade guide). However, this method requires the dentist to compare a plurality of color guides with the patient's teeth one by one, thereby significantly lowering work efficiency. Additionally, since this method heavily relies on the dentist's clinical experiences, it may lead to various human errors.

As an alternative method, digital equipment was used to acquire the color of the patient's teeth directly or by capturing an image of the patient's teeth, and then the color of the dental prostheses was determined. However, this method is vulnerable to noise (e.g., external light caused by scan conditions, etc.), significantly reducing the reliability of the process for determining the color of the dental prostheses.

### SUMMARY

The present disclosure provides a technology capable of determining the color of a patient's teeth with high reliability.

The present disclosure also provides a technology capable of simultaneously processing scanning the patient's oral cavity and determining the color of the patient's teeth using an intraoral scanner for scanning the patient's oral cavity.

The present disclosure also provides a technology capable of harmonizing objective information on determining the color of the patient's teeth with the clinical experiences of the denti st.

The present disclosure also provides a technology for continuously using an artificial intelligence model to determine the color of the patient's teeth and continuously obtaining learning data, thereby enhancing the color classification performance of the artificial intelligence model.

The present disclosure also provides a technology capable of generating initial training data for an artificial intelligence model designed to determine the color of the patient's teeth.

The present disclosure also provides a technology for determining the color of the patient's teeth and transmitting the color to the dental laboratory for the production of dental prostheses, so that dental prostheses may be produced to naturally blend with the patient's teeth.

The technical problems of the present disclosure are not limited to the aforementioned technical problems, and other technical problems not mentioned will be clearly understood by those skilled in the art to which the present disclosure pertains from the descriptions in the specification.

A method performed by an electronic device according to the present disclosure may include generating a three-dimensional image of an object based on a two-dimensional image set of the object obtained from an intraoral scanner, the object including a plurality of unit objects, based on a user's selection for a first point of the three-dimensional image, extracting a first color information set corresponding to a first region including the first point, using an artificial intelligence model configured to output a color similarity based on an input of the first color information set, and determining representative color information of a first unit object corresponding to the first point based on a magnitude of the color similarity.

In an embodiment, the method may further include transmitting the representative color information of the first unit object to an external terminal of the electronic device.

In an embodiment, the transmitting representative color information to the external terminal may include transmitting information about the first region including the first point to the external terminal.

In an embodiment, the extracting the first color information set corresponding to the first region may include determining the first region including a plurality of points within a reference distance from the first point.

In an embodiment, the three-dimensional image may include a valid region and an exceptional region and the extracting the first color information set corresponding to the first region may include, if the first region includes the exceptional region, extracting the first color information set corresponding to the first region with the exceptional region excluded.

In an embodiment, the extracting the first color information set corresponding to the first region may include identifying a first two-dimensional image set corresponding to at least some of a plurality of points included in the first region, the first two-dimensional image set being a sub-set of the two-dimensional image set and extracting a color code of a pixel of a two-dimensional image included in the first two-dimensional image set.

In an embodiment, the first two-dimensional image set may include only two-dimensional images used to generate the three-dimensional image.

In an embodiment, the extracting the color code of the pixel of the two-dimensional image included in the first two-dimensional image set may include determining an extraction order of two-dimensional images included in the first two-dimensional image set based on a scan order and extracting the color code of the pixel of the two-dimensional image included in the first two-dimensional image set based on the extraction order.

In an embodiment, the first color information set may include one or more pieces of first color information having a reference size and the extracting the first color information set corresponding to the first region may further include generating the first color information based on the reference size.

In an embodiment, the extracting the first color information set corresponding to the first region may further include converting the extracted color code into an HSV (hue, saturation, value) code.

In an embodiment, the first color information set may include N pieces (where N is a natural number) of first color information and the using the artificial intelligence model may include calculating sub-color similarities between respective reference colors included in a reference color group and the respective ones of the N pieces of first color information included in the first color information set and calculating the color similarity based on the N sub-color similarities.

In an embodiment, learning data of the artificial intelligence model may include a pair of a first label indicating a first reference color included in a reference color group and a first scan result of a first model corresponding to the first reference color.

In an embodiment, the learning data of the artificial intelligence model may further include a pair of the first label and a second scan result of the first model and second scan conditions of the second scan result may be at least partially different from first scan conditions of the first scan result.

In an embodiment, the determining the recommended color information may include including a reference color whose magnitude of the color similarity exceeds a reference value in the recommended color information.

In an embodiment, the determining the representative color information may include the steps of determining M (where M is a natural number) pieces of recommended color information based on the magnitude of color similarity and determining the representative color information based on a user's selection of the recommended color information.

In an embodiment, the determining the M pieces of recommended color information may include the step of excluding recommended color information with a magnitude of color similarity equal to or less than a reference value.

An electronic device according to the present disclosure may include a communication circuit configured to communicate with a network, a processor configured to execute a computer program including one or more instructions, and a memory configured to load the computer program, wherein the computer program may include an instruction for generating a three-dimensional image of an object based on a two-dimensional image set of the object obtained from an intraoral scanner, the object including a plurality of unit objects, an instruction for extracting, based on a user's selection for a first point of the three-dimensional image, a first color information set corresponding to a first region including the first point, an instruction for using an artificial intelligence model configured to output a color similarity based on an input of the first color information set, and an instruction for determining representative color information of a first unit object corresponding to the first point based on a magnitude of the color similarity.

A non-transitory computer-readable recording medium according to the present disclosure having a recorded computer program to be executed by a processor, the computer program including an instruction for generating a three-dimensional image of an object based on a two-dimensional image set of the object obtained from an intraoral scanner, the object including a plurality of unit objects, an instruction for extracting, based on a user's selection for a first point of the three-dimensional image, a first color information set corresponding to a first region including the first point, an instruction for using an artificial intelligence model configured to output a color similarity based on an input of the first color information set, and an instruction for determining representative color information of a first unit object corresponding to the first point based on a magnitude of the color similarity.

According to the present disclosure, it is possible to determine the color of a patient's teeth with high reliability.

According to the present disclosure, it is possible to simultaneously process scanning the patient's oral cavity and determining the color of the patient's teeth using an intraoral scanner for scanning the patient's oral cavity.

According to the present disclosure, it is possible to harmonize objective information on determining the color of the patient's teeth with the clinical experiences of the dentist.

According to the present disclosure, it is possible to enhance the color classification performance of an artificial intelligence model by continuously using the artificial intelligence model to determine the color of the patient's teeth and continuously obtaining learning data.

According to the present disclosure, it is possible to generate initial training data for an artificial intelligence model designed to determine the color of the patient's teeth.

According to the present disclosure, it is possible to produce dental prostheses that naturally blend with the patient's teeth by determining the color of the patient's teeth and transmitting the color to the dental laboratory for the production of dental prostheses.

The effects of the technical concepts of the present disclosure are not limited to the aforementioned effects, and other effects not mentioned will be clearly understood by those skilled in the art to which the present disclosure pertains from the descriptions in the specification.

### BRIEF DESCRIPTION OF DRAWINGS

FIG. 1 illustrates a scanning environment according to an embodiment of the present disclosure.
FIG. 2 illustrates a block diagram illustrating an electronic device and an intraoral scanner according to an embodiment of the present disclosure.
FIG. 3 illustrates an intraoral scanner according to an embodiment of the present disclosure.
FIG. 4 illustrates a diagram illustrating an operation of generating a three-dimensional image of the patient's oral cavity, which may be referenced in various embodiments of the present disclosure.
FIG. 5 illustrates a shade guide that can be referenced in various embodiments of the present disclosure.
FIG. 6 illustrates a flowchart illustrating a method according to an embodiment of the present disclosure.
FIG. 7 illustrates a flowchart specifically illustrating the operation of extracting a color information set described with reference to FIG. 6.
FIG. 8 illustrates a flowchart specifically illustrating the operation of extracting color codes described with reference to FIG. 7.
FIG. 9 illustrates a flowchart specifically illustrating the operation of using an artificial intelligence model described with reference to FIG. 6.
FIG. 10 illustrates a diagram illustrating a learning operation of an artificial intelligence model, which may be referenced in various embodiments of the present disclosure.
FIGS. 11 and 12 are diagrams illustrating an operation of calculating color similarity based on an artificial intelligence model, which may be referenced in various embodiments of the present disclosure.
FIG. 13 illustrates a flowchart specifically illustrating the operation of determining representative color information described with reference to FIG. 6.
FIG. 14 illustrates a flowchart illustrating a method according to an embodiment of the present disclosure.
FIGS. 15 to 18 illustrate screens of a program to which a method according to an embodiment of the present disclosure is applied.

### DETAILED DESCRIPTION

Various embodiments of the present disclosure are illustrated for describing the technical spirit of the present disclosure and are not intended to limit the present disclosure to specific embodiments. The technical spirit of the present disclosure includes various modifications, equivalents, and alternatives of the respective embodiments described in the present disclosure, and embodiments resulting from combinations of all or some of the respective embodiments. In addition, the scope of the technical spirit of the present disclosure is not limited to the various embodiments described below or to the detailed descriptions thereof.

All technical or scientific terms used herein have meanings that are generally understood by a person having ordinary knowledge in the art to which the present disclosure pertains, unless otherwise specified.

The expressions "include", "may include", "provided with", "may be provided with", "have", "may have", and the like used herein indicate the presence of relevant features (e.g., functions, operations, or components) and do not exclude the presence of other additional features. That is, such expressions should be understood as open-ended terms connoting the possibility of inclusion of other embodiments.

A singular expression used herein may include meanings of plurality, unless otherwise mentioned, and the same is applied to a singular expression stated in the claims.

The terms "first", "second", etc. used herein are used to identify a plurality of components from one another, unless otherwise mentioned, and are not intended to limit the order or importance of the relevant components.

The expressions "A, B, and C," "A, B, or C," "at least one of A, B, and C," or "at least one of A, B, or C," as used in the present disclosure may indicate each of the listed items or all possible combinations of the listed items. For example, "at least one of A or B" may refer to (1) at least one A, (2) at least one B, or (3) at least one A and at least one B.

The expression "based on" used herein is used to describe one or more factors that influences a decision, an action of judgment, or an operation described in a phrase or sentence including the relevant expression, and this expression does not exclude additional factor influencing the decision, the action of judgment or the operation.

The expression that a certain component (e.g., first component) is "coupled to" or "connected to" another component (e.g., second component), which is used herein, may indicate that the certain component is coupled or connected directly to the other component or that the certain component is coupled or connected to the other component via another component (e.g., third component).

The expression "configured to" as used in the present disclosure may indicate, depending on the context, "set to", "having the ability to", "modified to", "made to", "capable of", or the like. This expression is not limited to the meaning of "specifically designed in hardware", and for example, a processor configured to perform a specific operation may indicate a generic purpose processor capable of performing the specific operation by executing software, or a special purpose computer that is structured through programming to perform the specific operation.

The term "artificial intelligence (AI)" as used in the present disclosure may refer to a technology for mimicking human learning, reasoning, or perceptual abilities and implementing the same as a computer. Such AI may broadly include concepts such as machine learning or symbolic logic.

Specifically, the artificial intelligence is able to analyze input data using machine learning algorithms, learn from the results of the analysis, and make judgments or predictions based on the results of the learning. Furthermore, technologies for mimicking the cognitive or judgmental functions of the human brain using machine learning algorithms may also be understood as a kind of AI. For example, it may include fields such as language understanding, visual understanding, reasoning/prediction, knowledge representation, or motion control.

The term "machine learning" as used in the present disclosure may indicate a process of training a neural network model using experiences of data processing. It may indicate that computer software improves its data processing capabilities on its own through such machine learning. Here, a neural network model is constructed by modeling the correlations between data, which may be expressed by a plurality of parameters. This neural network model extracts and analyzes features from given data to derive correlations between the data, and this process is repeated to optimize the parameters of the neural network model, which is called machine learning. For example, the neural network model may learn the mapping (correlation) between input and output for data given as input-output pairs. Alternatively, even when only input data is given, the neural network model may learn the correlation by deriving the regularity between the given data.

The terms "artificial intelligence learning model," "machine learning model," or "neural network model" as used in the present disclosure may be designed to implement the human brain structure on a computer and may include a plurality of network nodes that mimic neurons of the human nervous system and have weights. Here, "the plurality of network nodes" may have interconnections with each other by mimicking the synaptic activity of neurons for transmitting and receiving signals through synapses. Specifically, in an artificial intelligence learning model, a plurality of network nodes may be located at different depths of layers and may exchange data according to convolutional connections. The artificial intelligence learning model may be, for example, an artificial neural network (ANN) model or a convolutional neural network (CNN) model, but the scope of the present disclosure is not limited to the above examples, and various known neural network models may be applied to the present disclosure.

Hereinafter, various embodiments of the present disclosure will be described with reference to the accompanying drawings. In the attached drawings and descriptions thereof, identical or substantially equivalent components may be assigned the same reference numerals. Additionally, in the following descriptions of various embodiments, the redundant descriptions of identical or corresponding components may be omitted, but this does not imply that such components are excluded from the embodiments.

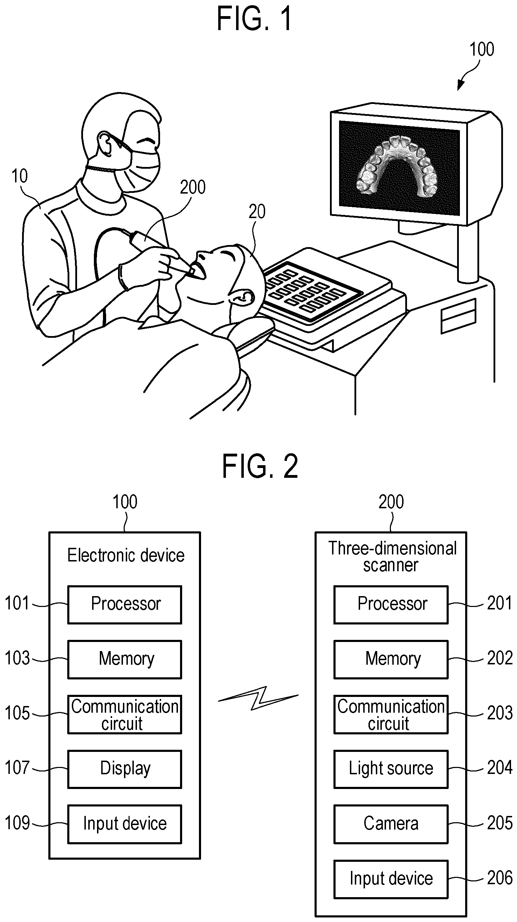

FIG. 1 illustrates a scanning environment according to an embodiment of the present disclosure. Specifically, the scanning environment shown in FIG. 1 is an environment for obtaining images of the oral cavity of a patient 20 using an intraoral scanner 200 according to an embodiment of the present disclosure. Here, the intraoral scanner 200 may be a dental medical device for obtaining images inside the oral cavity of the patient 20.

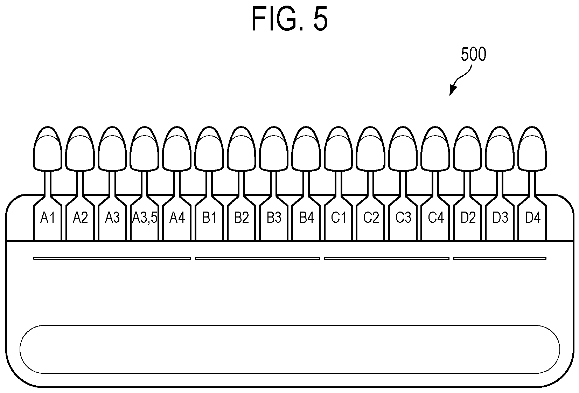

As shown in FIG. 1, a user 10 (e.g., a dentist or dental hygienist) may scan the oral cavity of a patient 20 for an image thereof using the intraoral scanner 200. In addition, in order to generate training data for an artificial intelligence model, the user 10 may also scan images of a shade guide (e.g., the shade guide 500 in FIG. 5) using the intraoral scanner 200.

Hereinafter, the operations of the components shown in FIG. 1 will be explained in detail.

The electronic device 100 shown in FIG. 1 may receive two-dimensional images of the oral cavity of the patient 20 from the intraoral scanner 200. For example, the electronic device 100 may receive 980 two-dimensional images per second from the intraoral scanner 200. If 14 two-dimensional images are defined as one frame, the electronic device 100 may receive 70 frames per second from the intraoral scanner 200.

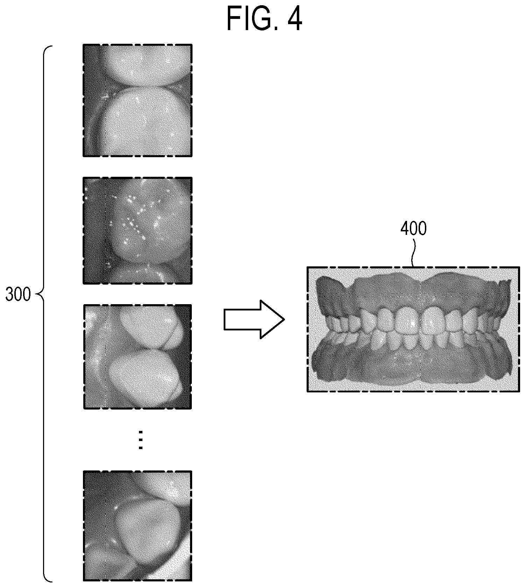

In addition, based on the received two-dimensional images of the oral cavity, the electronic device 100 may structure the internal structure of the oral cavity in three dimensions to generate a three-dimensional image of the oral cavity. The operation of generating this three-dimensional image will be described later with reference to FIG. 4. The three-dimensional image of the oral cavity generated by the electronic device 100 may be displayed to the user 10 or the patient 20 through a display of the electronic device 100. At this time, the user 10 may refer to the three-dimensional image displayed on the electronic device 100 to provide appropriate medical services to the patient 20.

In an embodiment, the electronic device 100 may receive a three-dimensional image of the oral cavity generated by the intraoral scanner 200 or an external device. That is, the scope of the present disclosure encompasses a three-dimensional image generated by the intraoral scanner 200 and a three-dimensional image generated by an external device other than the electronic device 100.

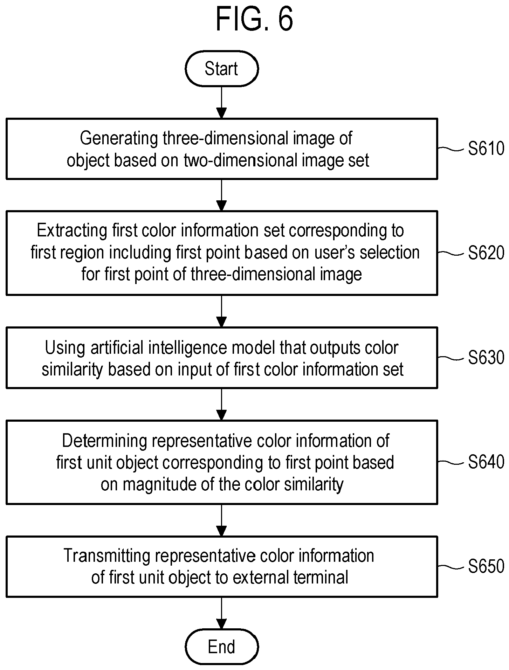

In addition, the electronic device 100 may determine representative color information of each of one or more teeth of the patient 20. This representative color information may be transmitted to a dental laboratory terminal later and used for the production of the prostheses of the patient 20. The operation of determining this representative color information will be described later with reference to FIG. 6 and subsequent drawings.

Furthermore, the electronic device 100 may communicate with a cloud server. In this case, the electronic device 100 may transmit two-dimensional images of the oral cavity of the patient 20 or three-dimensional images of the oral cavity to the cloud server, and the cloud server may store the two-dimensional images of the oral cavity of the patient 20 or three-dimensional images of the oral cavity received from the electronic device 100.

The electronic device 100 may also communicate with a dental laboratory terminal. In this case, the electronic device 100 may determine representative color information for each of one or more teeth of the patient 20 and transmit the representative color information to the dental laboratory terminal, thereby requesting the production of prostheses corresponding to the representative color information.

The electronic device 100 described above may be implemented as a computing device, and an example of such a computing device will be described later with reference to FIG. 2.

The intraoral scanner 200 shown in FIG. 1 may have a form that allows insertion into and removal from the oral cavity and may be a handheld scanner that enables the user 10 to freely adjust the scanning distance and angle.

This intraoral scanner 200 may be inserted into the oral cavity and scan the interior of the oral cavity in a non-contact manner to obtain images of the oral cavity. The images of the oral cavity may include at least one tooth, gingiva, and/or artificial structure that is insertable into the oral cavity (e.g., orthodontic appliances including brackets and wires, implants, dentures, orthodontic auxiliary tools inserted into the oral cavity, etc.). Specifically, the intraoral scanner 200 may irradiate the oral cavity of the patient 20 with light using a light source and receive the light reflected from the oral cavity of the patient 20 through a camera.

In addition, based on the information received through the camera, the intraoral scanner 200 may obtain surface images of the oral cavity of the patient 20 as two-dimensional images. Here, the surface images of the oral cavity of the patient 20 may include at least one of teeth, gingiva, artificial structures, cheeks, tongue, or lips of the patient 20.

The intraoral scanner 200 described above may be implemented as a computing device, and an example of such a computing device will be described in detail below with reference to FIG. 2.

FIG. 2 illustrates a block diagram illustrating an electronic device 100 and an intraoral scanner 200 according to an embodiment of the present disclosure. The block diagram in FIG. 2 merely shows a preferable embodiment to attain the objectives of the present disclosure, and some components may be added or deleted as needed. In addition, the components of the block diagrams shown in FIG. 2 represent functionally distinguished functional elements, and it should be noted that a plurality of different components may be implemented in an integrated form in an actual physical environment. Hereinafter, respective components shown in the block diagram will be described in detail.

The electronic device 100 shown in FIG. 2 may include one or more processors 101, one or more memories 103, a communication circuit 105, a display 107, or an input device 109. As previously described, at least one of the components included in the electronic device 100 may be omitted, or other components may be added to the electronic device 100. In addition, additionally or alternatively, some components may be integrated or may be implemented as single or a plurality of entities. At least some of the components in the electronic device 100 may be interconnected through buses, GPIOs (general-purpose input/outputs), SPIs (serial peripheral interfaces), MIPIs (mobile industry processor interfaces), or the like to exchange data or signals.

One or more processors 101 of the electronic device 100 may perform computations or data processing related to the control or communication of the respective components (e.g., the memory 103) of the electronic device 100. One or more processors 101 may be operatively connected to, for example, the components of the electronic device 100. In addition, one or more processors 101 may load commands or data received from other components of the electronic device 100 into one or more memories 103, process the commands or data stored in one or more memories 103, and store the result data.

One or more memories 103 of the electronic device 100 may store various data, commands, or information. As a specific example, one or more memories 103 may store instructions, as computer programs, for the operation of the processors 101. In addition, one or more memories 103 may store artificial intelligence models built according to machine learning algorithms. In addition, one or more memories 103 may store data (e.g., two-dimensional images of the oral cavity or three-dimensional images of the oral cavity) received from the intraoral scanner 200.

The communication circuit 105 of the electronic device 100 may establish a wired or wireless communication channel with an external device, and transmit and receive various data to and from the external device. In an embodiment, the communication circuit 105 may include at least one port for connecting to the external device with a wired cable in order to communicate with the external device through a wire. In this case, the communication circuit 105 may perform communication with the external device through a wired connection using at least one port. In another embodiment, the communication circuit 105 may be configured to include a cellular communication module and to be connected to a cellular network (e.g., 3G, LTE, 5G, Wibro, or WiMAx). In another embodiment, the communication circuit 105 may include a short-range communication module, and transmit and receive data to and from an external device using short-range communication (e.g., Wi-Fi, Bluetooth, Bluetooth Low Energy (BLE), or UWB). In another embodiment, the communication circuit 105 may include a contactless communication module for contactless communication. Here, the contactless communication may include at least one contactless proximity communication technology such as NFC (near-field communication), RFID (radio frequency identification) communication, or MST (magnetic secure transmission) communication. In addition to the various examples described above, the electronic device 100 may be implemented in various known ways for communication with an external device, and it should be noted that the scope of the present disclosure is not limited to the examples described above.

The display 107 of the electronic device 100 may display various screens under the control of the processor 101. Here, based on the control of the processor 101, the two-dimensional image of the oral cavity of the patient 20, which is received from the intraoral scanner 200, or the three-dimensional image of the oral cavity in which the internal structure of the oral cavity is modeled in three dimensions may be displayed through the display 107. In this case, for example, a web browser or a dedicated application may be installed on the electronic device 100 in order to display the two-dimensional image or three-dimensional image of the oral cavity on the display 107. In an embodiment, the aforementioned web browser or dedicated application may be implemented to provide the user 10 with editing, saving, or deleting functions for the two-dimensional image or three-dimensional image of the oral cavity through a user interface.

The input device 109 of the electronic device 100 may receive commands or data to be used by the components (e.g., the processor 101) of the electronic device 100 from the outside (e.g., the user) of the electronic device 100. The input device 109 may include, for example, a microphone, mouse, or keyboard. In an embodiment, the input device 109 may be implemented as a touch sensor panel that is connected to the display 107 and is able to recognize the contact or proximity of various external objects. However, the scope of the present disclosure is not limited to the aforementioned examples, and various known input devices 109 may be included within the scope of the present disclosure for the convenience of the user.

The intraoral scanner 200 shown in FIG. 2 may include a processor 201, a memory 202, a communication circuit 203, a light source 204, a camera 205, or an input device 206. As described above, at least one of the components included in the intraoral scanner 200 may be omitted, or other components may be added to the intraoral scanner 200. In addition, additionally or alternatively, some components may be integrated or may be implemented as single or a plurality of entities. At least some of the components in the intraoral scanner 200 may be interconnected through buses, GPIOs (general-purpose input/outputs), SPIs (serial peripheral interfaces), MIPIs (mobile industry processor interfaces), or the like to exchange data or signals.

The processor 201 of the intraoral scanner 200 may perform computations or data processing related to the control or communication of the respective of the intraoral scanner 200, and may be operatively connected to the components of the intraoral scanner 200. In addition, the processors 201 may load commands or data received from other components of the intraoral scanner 200 into the memory 202, process the commands or data stored in the memory 202, and store the result data.

The memory 202 of the intraoral scanner 200 may store instructions for the operations of the processor 201 described above.

The communication circuit 203 of the intraoral scanner 200 may establish wired or wireless communication channels with external devices (e.g., the electronic device 100) and transmit and receive various data to and from the external devices. In an embodiment, the communication circuit 203 may include at least one port for connecting to the external device with a wired cable in order to communicate with the external device through a wire. In this case, the communication circuit 203 may perform communication with the external device through a wired connection using at least one port. In another embodiment, the communication circuit 203 may be configured to include a cellular communication module and to be connected to a cellular network (e.g., 3G, LTE, 5G, Wibro, or WiMAx). In another embodiment, the communication circuit 203 may include a short-range communication module, and transmit and receive data to and from an external device using short-range communication (e.g., Wi-Fi, Bluetooth, Bluetooth Low Energy (BLE), or UWB). In another embodiment, the communication circuit 203 may include a contactless communication module for contactless communication. Here, the contactless communication may include at least one contactless proximity communication technology such as NFC (near-field communication), RFID (radio frequency identification) communication, or MST (magnetic secure transmission) communication. In addition to the various examples described above, the intraoral scanner 200 may be implemented in various known ways for communication with an external device, and it should be noted that the scope of the present disclosure is not limited to the examples described above.

The light source 204 of the intraoral scanner 200 may emit light to the oral cavity of the patient 20. For example, the light emitted from the light source 204 may be structured light with a predetermined pattern (e.g., a stripe pattern with continuous lines of different colors). Here, the pattern of the structured light may be generated using, for example, a pattern mask or a DMD (digital micro-mirror device), but it is not limited thereto.

The camera 205 of the intraoral scanner 200 may receive the light reflected from the oral cavity of the patient 20 to obtain images of the oral cavity of the patient 20. Here, the camera 205 may include a left camera corresponding to the left eye view and a right camera corresponding to the right eye view to produce a three-dimensional image according to optical triangulation. In addition, the camera 205 may include at least one image sensor, such as a CCD sensor or a CMOS sensor.

The input device 206 of the intraoral scanner 200 may receive user inputs to control the intraoral scanner 200. For example, the input device 206 may include a button for receiving push operations from the user 10, a touch panel for detecting a touch of the user 10, or a voice recognition device including a microphone. Here, the user 10 may control the start or stop of scanning using the input device 206.

Describing the operation of the intraoral scanner 200 controlled through the input device 206 in more detail, the intraoral scanner 200 may receive a user input to start scanning through the input device 206 of the intraoral scanner 200 or the input device 206 of the electronic device 100, and start scanning according to processing by the processor 201 of the intraoral scanner 200 or the processor 101 of the electronic device 100. Here, when the user 10 scans the inside of the oral cavity of the patient 20 using the intraoral scanner 200, the intraoral scanner 200 may generate two-dimensional images of the oral cavity of the patient 20 and transmit the two-dimensional images of the oral cavity of the patient 20 to the electronic device 100 in real-time. In this case, the electronic device 100 may display the received two-dimensional images of the oral cavity of the patient 20 through the display 107. In addition, the electronic device 100 may generate (construct) a three-dimensional image of the oral cavity of the patient 20 based on the two-dimensional images of the oral cavity of the patient 20 and display the three-dimensional image of the oral cavity through the display 107. At this time, the electronic device 100 may display the three-dimensional image being generated in real-time on the display 107.

The sensor module 207 of the intraoral scanner 200 may detect the operating state of the intraoral scanner 200 or the external environmental state (e.g., the user's action) and generate an electrical signal corresponding to the detected state. The sensor module 207 may include at least one of a gyro sensor, an accelerometer, a gesture sensor, a proximity sensor, or an infrared sensor. Here, the user 10 may control the start or stop of scanning using the sensor module 207. For example, when the user 10 holds and moves the intraoral scanner 200, the intraoral scanner 200 may control the processor 201 to start a scanning operation when the angular velocity measured by the sensor module 207 exceeds a setting value.

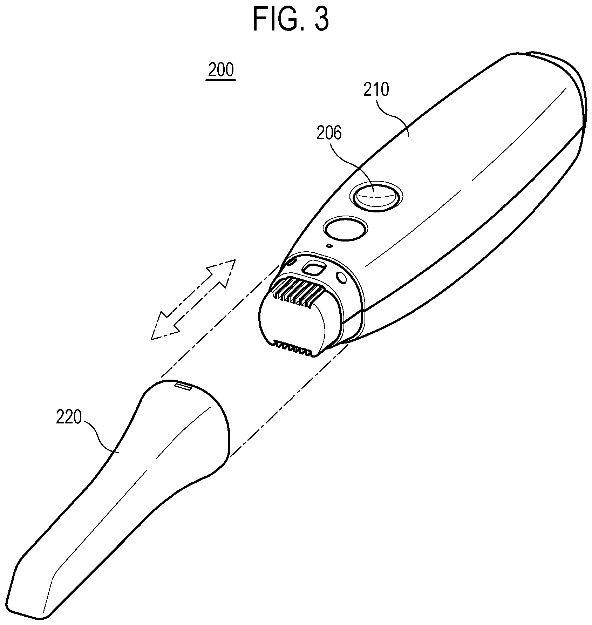

Hereinafter, the intraoral scanner 200 described above will be described in more detail with reference to FIG. 3. FIG. 3 illustrates an intraoral scanner 200 according to an embodiment of the present disclosure.

The intraoral scanner 200 shown in FIG. 3 may include a main body 210 and a probe tip 220. Here, the main body 210 of the intraoral scanner 200 may be shaped to be easily gripped and used by hands of the user 10, and the probe tip 220 may be shaped for easy insertion into and removal from the oral cavity of the patient 20. In addition, the main body 210 may be coupled to and detached from the probe tip 220. Furthermore, the components of the intraoral scanner 200 described in FIG. 2 may be disposed inside the main body 210. The main body 210 may have an opening at one end through which light output from the light source 204 may be emitted to the patient 20. The light emitted through the opening may be reflected by the patient 20 and may enter back through the opening. Here, the reflected light entering through the opening may be captured by the camera to generate images of the patient 20. In addition, the user 10 may start scanning using the input device 206 (e.g., a button) of the intraoral scanner 200. For example, when the user 10 touches or presses the input device 206, light from the light source 204 may be emitted toward the patient 20.

FIG. 4 illustrates a diagram illustrating an operation of generating a three-dimensional image of the oral cavity of a patient 20, which may be referenced in various embodiments of the present disclosure. As described above, the user 10 may scan the inside of the oral cavity of the patient 20 while moving the intraoral scanner 200, and in this case, the intraoral scanner 200 may obtain a two-dimensional image set 300 of the oral cavity of the patient 20. For example, the intraoral scanner 200 may obtain a two-dimensional image of a region including the front teeth of the patient 20, a two-dimensional image of a region including the molars of the patient 20, and the like. At this time, the intraoral scanner 200 may transmit the obtained two-dimensional image set 300 to the electronic device 100.

The electronic device 100 may convert each of the two-dimensional images included in the two-dimensional image set 300 for the oral cavity of the patient 20 into a set of multiple points having three-dimensional coordinate values using the two-dimensional image set 300. For example, the electronic device 100 may convert the two-dimensional images included in the two-dimensional image set 300 into a point cloud, which is a set of data points having three-dimensional coordinate values. Here, the point cloud, which is a three-dimensional coordinate value based on the two-dimensional image set 300, may be stored as raw data on the oral cavity of the patient 20. In addition, the electronic device 100 may generate a three-dimensional image 400 for the oral cavity of the patient 20 by aligning the point cloud, which is a set of data points having three-dimensional coordinate values.

In an embodiment, the electronic device 100 may reconfigure (rebuild) a three-dimensional image 400 of the oral cavity. For example, the electronic device 100 may reconfigure a plurality of points by merging point clouds stored as raw data using a Poisson algorithm and convert them into a closed three-dimensional surface, thereby reconfiguring the three-dimensional image 400 for the oral cavity of the patient 20. However, unlike this example, it should be noted that raw data may be processed in various known ways and that the scope of the present disclosure encompasses any method of reconfiguring the three-dimensional image 400 of the oral cavity.

FIG. 5 illustrates a shade guide 500 that may be referenced in various embodiments of the present disclosure. Here, the shade guide 500 may be a set of models representing reference colors. Specifically, the shade guide 500 may include a first model representing a first reference color and a second model representing a second reference color.

The shade guide 500 may be, for example, the VITA classical A1-D4^{®} shade guide. The VITA classical A1-D4^{®} shade guide may be a set of 16 models respectively representing 16 reference colors. As another example, the shade guide 500 may be the VITA SYSTEM 3D-MASTER^{®}. In addition to the examples described above, a set of various models may be the shade guide 500, and any set of models representing reference colors for naturally expressing a patient's teeth may be included within the scope of the present disclosure.

In the present disclosure, the set of reference colors expressed in the shade guide 500 in FIG. 5 may be referred to as a reference color group. The reference color group may be used as the standard for classifying the colors of the patient's teeth, i.e., as labels, in the method described with reference to FIG. 6 and subsequent drawings.

Hereinafter, a method according to various embodiments of the present disclosure will be described in detail. Although the operations are shown in a specific order in the following drawings, it should be noted that the operations do not necessarily have to be performed in the specific order or sequentially as shown in the drawing, nor do all the shown operations need to be performed to achieve the desired results.

In addition, the operations of the method described with reference to the following drawings may be performed by a computing device. In other words, the operations of the method may be implemented as one or more instructions executed by the processor of a computing device. Although all operations included in the method may be executed by a single physical computing device, a first operation of the method may be performed by a first computing device, and a second operation may be performed by a second computing device.

Hereinafter, it will be assumed that the operations of the aforementioned method are performed by the electronic device 100 shown in FIG. 1. However, for convenience of description, the subject of the operations included in the method may be omitted, but unless otherwise specified in context, the operations should be interpreted as being performed by the electronic device 100.

FIG. 6 illustrates a flowchart illustrating a method according to an embodiment of the present disclosure. The method shown in FIG. 6 may include a series of operations to determine representative color information of the teeth of a patient 20 teeth and request the production of prostheses. The operations shown in FIG. 6 will be described below.

Based on a two-dimensional image set, a three-dimensional image of an object may be generated (S610).

The two-dimensional image set may be a set of two-dimensional images obtained from the intraoral scanner 200. Each two-dimensional image included in the two-dimensional image set may include a two-dimensional image of at least a portion of the object (e.g., the oral cavity of the patient 20). Since the object may include a plurality of unit objects (e.g., respective teeth), each two-dimensional image included in the two-dimensional image set may include a two-dimensional image of at least a portion of the unit object.

The three-dimensional image may be an image generated by processing the two-dimensional image set. In generating such a three-dimensional image, at least some of the two-dimensional images included in the two-dimensional image set may be used. In other words, at least some of the two-dimensional images included in the two-dimensional image set may not be used in generating the three-dimensional image. The detailed description of generating the three-dimensional image may be understood with reference to the description of FIG. 4 above.

Based on a selection of the user 10 for a first point of the three-dimensional image, a first color information set corresponding to a first region including the first point may be extracted (S620).

The first point is determined based on the selection of the user 10 and may be any point on the three-dimensional image. To select such a first point, the electronic device 100 may display the three-dimensional image on the display 107 or implement a user interface for receiving a user's selection through the input device 109.

The selection of the user 10 may be an input for selecting any point on the three-dimensional image. The selection of the user 10 may be, for example, a click on any point on the three-dimensional image, but it is not limited thereto, and any operation defined to select a point on the three-dimensional image may fall within the scope of the present disclosure.

The first region may be any region on the three-dimensional image dynamically determined according to the determination of the first point. In an embodiment, the first region may be a region that includes a plurality of points within a reference distance from the first point. The reference distance is a factor for determining the radius of the first region and may be, for example, 2mm. According to this example, the first region may be determined to include all points within 2mm of the first point. The reference distance may correspond to the distance on the two-dimensional region displayed on the display 107 of the electronic device 100, and accordingly, the first region may be displayed as a precise circle having the center of the first point and the radius of the reference distance on the display 107 of the electronic device 100, but the first region projected on the three-dimensional image may not be an exact circle.

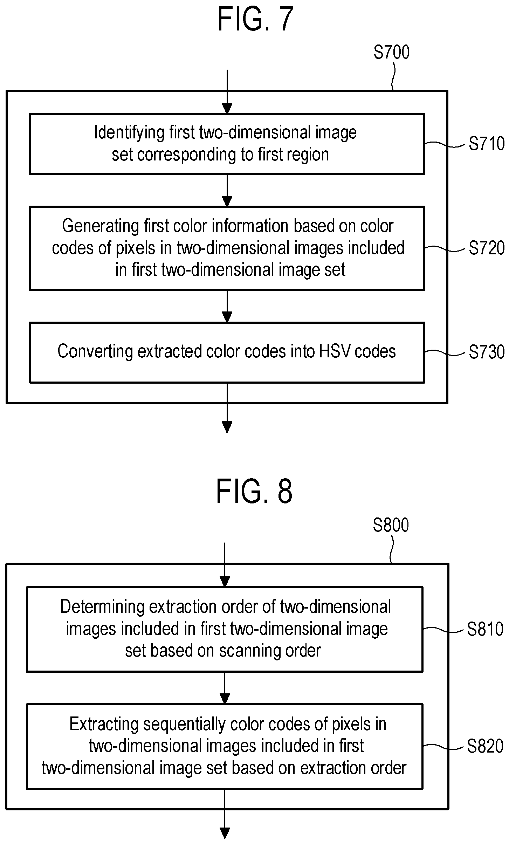

The first color information set may be a set of first color information. In addition, the first color information may be information generated from color codes (e.g., RGB codes, HSV codes, etc.) of pixels included in the two-dimensional images related to the first region. That is, the first color information set may be a set of color codes related to the first region. The generation of the first color information and the first color information set will be described later with reference to FIGS. 7 to 8.

According to this operation, information about the color of the point (i.e., the first point) on the three-dimensional image, which is intended by the user 10, may be extracted as a set of color codes related to the region (i.e., the first region) determined based on that point. Thus, a set of color codes related to the tooth intended by the user 10 may be extracted.

In connection with determining the first region, in an embodiment, if the first region includes an exceptional region, a first color information set corresponding to the first region with the exceptional region excluded may be extracted. The three-dimensional image may include a valid region (e.g., the tooth region) of interest to the user 10 and an exceptional region (e.g., the gingiva region) that is not of interest. According to this embodiment, based on the above, even if the first region includes an exceptional region that is not of interest to the user 10, a first color information set, in which the color codes related to the exceptional region are excluded, may be extracted. Therefore, information about the color of the gingiva, which is not related to the color of the teeth, may be excluded, so the representative color information of the teeth may be determined with high reliability according to the operations described below. In addition, to implement this embodiment, techniques for distinguishing the tooth region from the gingiva region on the two-dimensional or three-dimensional image may be referenced. The tooth region, expressed in white, and the gingiva region, expressed in red, may be distinguished based on color differences, and for example, an artificial intelligence model optimized for distinguishing the tooth region and the gingiva region may be referenced in the present disclosure. In an embodiment, even if two or more teeth are included in the first region (e.g., if the first point is selected at the position near the boundary between two teeth), the color codes related to the region of the tooth, which does not include the first point, may also be extracted as the first color information set. In this case, computing resources consumed to distinguish the white-colored teeth may be reduced. In addition, since a point on another tooth closer to the first point is more likely to be similar in color to the first point than a point on the same tooth relatively far from the first point, the accurate first color information set may be extracted for the first point intended by the user. In another embodiment, if two or more teeth are included in the first region (e.g., if the first point is selected at the position near the boundary between two teeth), a first color information set, in which color codes related to the region of the tooth that does not include the first point are excluded, may be extracted. In this case, the first color information set may be extracted to match the color of the tooth intended by the user.

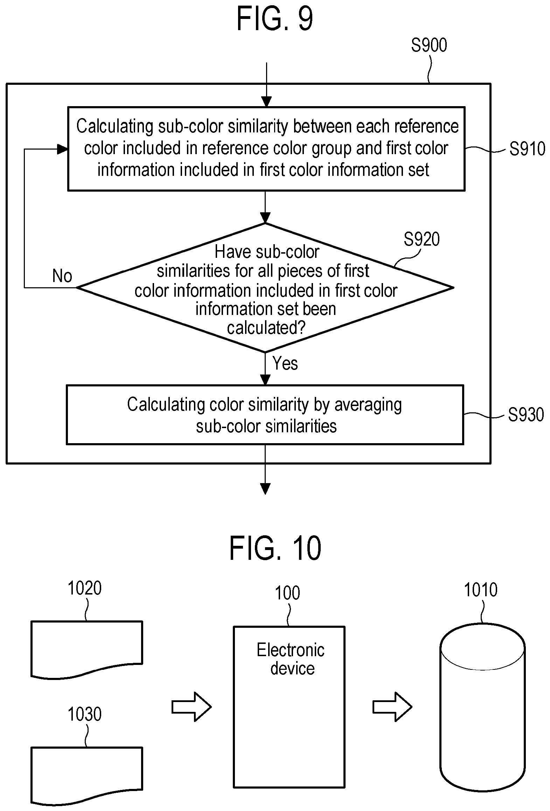

Based on the input of the first color information set, an artificial intelligence model that outputs a color similarity may be used (S630). Such an artificial intelligence model may refer to, for example, a classification model that classifies the first color information set (i.e., input color information) as one of the reference colors included in the reference color group. This will be described later with reference to FIGS. 10 to 12.

The color similarity may be a value indicating the degree of similarity between the first color information set and the reference color. For example, if the color similarity is higher, it may be understood as a reference color relatively similar to the first color information set, and if the color similarity is lower, it may be understood as a reference color relatively dissimilar to the first color information set.

In connection with the color similarity, in an embodiment, since the first color information set is a set of first color information, the color similarity may be calculated based on the similarity (i.e., sub-color similarity) between each of one or more pieces of the first color information and the reference color (e.g., by calculating the average of the sub-color similarities). This will be described later with reference to FIG. 9.

Since the reference color group is a set of reference colors, according to this operation, a color similarity between the first reference color and the first color information set may be calculated, and a color similarity between the second reference color and the first color information set may be calculated. In other words, according to this operation, the color similarity of the first color information set may be calculated with respect to each reference color included in the reference color group. In an embodiment, the color similarity may be calculated such that a sum of the color similarities between all reference colors in the reference color group and the first color information set reaches a certain value (e.g., 1).

Based on the magnitude of the color similarity, representative color information of a first unit object corresponding to the first point may be determined (S640).

The first unit object may be a unit object included in the object and may be a unit object (e.g., tooth) that includes the first point. In other words, the first unit object may be a unit object of interest to the user 10.

The representative color information may be color information representing the first unit object. This representative color information may be transmitted to an external terminal (e.g., a dental laboratory terminal) according to the operation described below, and may be used as information for determining the color of the prosthesis.

According to this operation, for example, since a reference color having the greatest magnitude of color similarity may be determined as the representative color information, a reference color that is estimated to be most similar to the actual color of the first unit object may be determined as the representative color information.

The representative color information of the first unit object may be transmitted to an external terminal (e.g., a dental laboratory terminal or a cloud server) (S650).

According to this operation, the representative color information determined corresponding to the first unit object may be dynamically transmitted to the external terminal, thereby initiating production of the prosthesis without a separate request for the production of the prosthesis.

For a specific example, the representative color information of the first unit object may be transmitted to the dental laboratory terminal. As another example, the representative color information of the first unit object may be transmitted to the dental laboratory terminal via the cloud server.

FIG. 7 illustrates a flowchart specifically illustrating the operation of extracting a color information set described with reference to FIG. 6. The method (S700) illustrated in FIG. 7 may include a series of operations related to the generation of the first color information and the generation of the first color information set as detailed operations of the extraction operation (S620) of the first color information set illustrated in FIG. 6. Hereinafter, the operations illustrated in FIG. 7 will be described.

A first two-dimensional image set corresponding to the first region may be identified (S710).

The first two-dimensional image set may be a sub-set of the two-dimensional image set received from the intraoral scanner 200. In other words, the first two-dimensional image set may be a set of two-dimensional images related to the first region among the two-dimensional image set received from the intraoral scanner 200.

According to this operation, the first two-dimensional image set may be identified by querying the point cloud of a plurality of points included in the first region.

In connection with the first two-dimensional image set, in an embodiment, the first two-dimensional image set may include only the two-dimensional images used in generating the three-dimensional image. Since only some of the two-dimensional images received from the intraoral scanner 200 may be used in generating the three-dimensional image, the first two-dimensional image set may include only the two-dimensional images used in generating the three-dimensional image. According to this embodiment, two-dimensional images, which are not used in generating the three-dimensional image, for example, images with a lot of noise, may be excluded.

Based on color codes of the pixels in the two-dimensional images included in the first two-dimensional image set, the first color information may be generated (S720).

According to this operation, the color codes of the pixels in the two-dimensional images related to the first region, especially those used in generating the three-dimensional image, may be extracted. The color codes extracted above may be used to generate the first color information.

In connection with the time of generating the first color information, in an embodiment, the first color information may be generated such that the number of extracted color codes becomes a reference size after all the pixels of the two-dimensional image are extracted as color codes. Here, the reference size may be a defined size of the first color information. For example, the reference size may be 128 x 128, which is 16,384. This reference size may be modified to be suitable as input data for the artificial intelligence model to be described later. According to this embodiment, the extracted color codes may be sequentially assigned as an item of the first color information in an arbitrary order (e.g., the scanning order of the two-dimensional images, the extraction order of the color codes, etc.). For example, if the reference size is 128 x 128, the first color code may be assigned to [1, 1] of the first color information, and the next color code may be assigned to [1, 2]. According to this order, the assignment may be performed up to [128, 128] of the first color information. If the assignment to [128, 128] is completed, the generation of the first color information (i.e., patch) may be completed. If there are remaining extracted color codes after the generation of the first color information is completed, additional first color information may be generated in the same manner as described above. In an embodiment, if the number of remaining extracted color codes is insufficient to generate a single piece of first color information (i.e., less than 16,384 in the above example), the remaining extracted color codes may be deleted.

In connection with the time of generating the first color information, in another embodiment, the first color information may be generated whenever the number of extractions of color codes based on the pixels of the two-dimensional images reaches the reference size. In other words, the extraction of color codes and the generation of the first color information may be performed in parallel. According to this embodiment, the sequentially extracted color codes may be sequentially assigned as an item of the first color information. For example, if the reference size is 128 × 128, the first extracted color code may be assigned to [1, 1] of the first color information, and the next color code may be assigned to [1, 2]. According to this order, the assignment may be performed up to [128, 128] of the first color information. If the assignment to [128, 128] is completed, the generation of the first color information (i.e., patch) may be completed. If there are remaining color codes, the technical idea described in the above embodiment may also be applied in the same manner to this embodiment.

In connection with the time of generating the first color information, various modifications may be made in addition to the embodiments described above. For example, the first color information may be generated such that the number of extracted color codes becomes the reference size after at least some of the pixels of the two-dimensional image are extracted as color codes. In other words, the time of generating the first color information may vary depending on the actual implementation case.

Regarding the extraction method of the color codes, in an embodiment, all the pixels of the two-dimensional image may be extracted as color codes. In another embodiment, at least some pixels of the two-dimensional image may not be extracted as color codes. In addition to the examples described above, any rule for extracting the color codes of the pixels related to the first region may fall within the scope of the present disclosure.

The extracted color codes may be converted to HSV (hue, saturation, value) codes (S730).

The HSV code may be a standard for expressing color. The HSV code may be a three-dimensional code that includes hue, saturation, and value as one coordinate.

According to the present operation, for example, color codes extracted as RGB codes may be converted to HSV codes. If the codes are extracted as HSV codes, this operation may be omitted.

In an embodiment, the time of converting to HSV codes may be any time after the extraction of color codes. For example, the color codes may be converted to HSV codes whenever they are extracted. As another example, all the color codes included in the first color information may be converted to HSV codes at once after the generation of the first color information is completed. As another example, all the color codes included in the first color information set may be converted to HSV codes at once after the generation of the first color information set is completed.

FIG. 8 illustrates a flowchart specifically illustrating the operation of extracting color codes described with reference to FIG. 7. The method (S800) shown in FIG. 8 may include details of the operation of generating the first color information (S720) shown in FIG. 7. The operations shown in FIG. 8 will be described below.

Based on the scanning order, the extraction order of the two-dimensional images included in the first two-dimensional image set may be determined (S810), and based on the extraction order, the color codes of the pixels in the two-dimensional images included in the first two-dimensional image set may be sequentially extracted (S820).

The scanning order may be the order in which the images were scanned by the intraoral scanner 200. In addition, the extraction order may be the order in which the color codes are extracted from the two-dimensional images included in the first two-dimensional image set. According to this operation, the two-dimensional images included in the first two-dimensional image set may be sequentially extracted corresponding to the scanning order.

FIG. 9 illustrates a flowchart specifically illustrating the operation of using an artificial intelligence model described with reference to FIG. 6. The method S900 shown in FIG. 9 may include details of the operation of using the artificial intelligence model (S630) shown in FIG. 6, and may include a series of operations for calculating the similarity between the reference color group and the first color information set using the artificial intelligence model. The operations shown in FIG. 9 will be described below.

A sub-color similarity between each reference color included in the reference color group and the first color information included in the first color information set may be calculated (S910).

The sub-color similarity may be a value indicating the degree of similarity between the first color information included in the first color information set and the reference color. Similar to the color similarity, for example, if the sub-color similarity is higher, it may be understood as a reference color relatively similar to the first color information, and if the sub-color similarity is lower, it may be understood as a reference color relatively dissimilar to the first color information.

According to this operation, the sub-color similarity between the first reference color and the first color information may be calculated, and the sub-color similarity between the second reference color and the first color information may be calculated. In other words, according to this operation, the sub-color similarity of the first color information may be calculated for each reference color included in the reference color group.

If the sub-color similarities for all pieces of first color information included in the first color information set have not been calculated (S920), a sub-color similarity between each reference color included in the reference color group and another piece of first color information included in the first color information set may be calculated (S910).

Since the first color information set may be a set of first color information, according to this operation, the sub-color similarities for all pieces of first color information included in the first color information set may be calculated. For example, if the first color information set includes N pieces (where N is a natural number) of first color information, N sub-color similarities corresponding to the N pieces of first color information may be calculated.

If the sub-color similarities for all pieces of first color information included in the first color information set have been calculated (S920), a color similarity may be calculated based on the sub-color similarities (S930).

For example, by averaging the calculated sub-color similarities, the color similarity for each reference color included in the reference color group may be calculated. For example, if the first color information set includes N pieces of first color information, the color similarity for the first reference color and the first color information set may be calculated by averaging N pieces of sub-color similarities of the first reference color and the first color information set, and the color similarity for the second reference color and the first color information set may be calculated by averaging N pieces of sub-color similarities of the second reference color and the first color information set. As another example, one or more of various methods for calculating color similarity based on sub-color similarity, such as summing the calculated sub-color similarities, normalizing after summing the same, or weighting the average thereof, may be used.

In this way, since the color similarity for the first color information set and each reference color included in the reference color group is calculated, the order of reference colors similar to the first color information set may be calculated as an objective indicator.

FIG. 10 illustrates a diagram illustrating a learning operation of an artificial intelligence model, which may be referenced in various embodiments of the present disclosure. Specifically,

FIG. 10 illustrates an example of constructing an artificial intelligence model 1010 using a first label 1020 indicating a first reference color included in a reference color group and a first scan result 1030 of a first model corresponding to the first reference color as learning data.

The first label 1020 may be a classification criterion, specifically, a label of supervised learning. For example, if the VITA classical A1-D4^{®} shade guide is used as a shade guide 500, "A1," "A2," or "D4" of the VITA classical A1-D4^{®} shade guide may be the first label 1020.

The first scan result 1030 may be a data set corresponding to the first label 1020 and may be a scan result of the first model having the first reference color corresponding to the first label. This first scan result 1030 may be generated in the same manner as the first color information described above. This first scan result 1030 may be generated as a patch, which is a single input unit of the artificial intelligence model 1010, to have a size of, for example, 128 x 128. As described above, by utilizing the scan result for the model having the reference color as learning data, a sufficient amount of learning data may be secured to learn the artificial intelligence model 1010.

According to FIG. 10, since the first label 1020 and the first scan result 1030 are input as a pair to the electronic device 100, the first label 1020 and the first scan result 1030 may be used for learning the artificial intelligence model 1010 stored in the electronic device 100. Specifically, since the first scan result 1030 is used as input data of the artificial intelligence model 1010 and since the first label 1020 is used as output data, the artificial intelligence model 1010 may be trained to classify an input of the first scan result 1030 into the first label 1020.

For example, if there is a reference color group including two reference colors, and if the color code included in the first scan result 1030 is two-dimensional, two weight sets for calculating the similarity of the reference colors may be adjusted such that the similarity of the reference color corresponding to the first label 1020 is calculated to be the greatest. Each of these two weight sets has two scalars. As another example, if there is a reference color group including 16 reference colors, and if the color code included in the first scan result 1030 is three-dimensional, 16 weight sets for calculating the similarity of the reference colors may be adjusted such that the similarity of the reference color corresponding to the first label 1020 is calculated to be the greatest. Each of these 16 weight sets has three scalars. The above-described machine learning may be performed in the electronic device 100 illustrated in FIG. 1, but it may also be performed externally and the learned artificial intelligence model 1010 may be embedded in the electronic device 100.

Here, the artificial intelligence model 1010 may perform various preprocessing on the color code included in the first scan result 1030, thereby converting the first scan result 1030 into a form suitable for a mathematical formula for calculating the similarity of the reference color. In an embodiment, the dimension of the first scan result 1030 may be reduced. For example, if the first scan result 1030 is a three-dimensional HSV code having a size of 2 x 2, the first scan result 1030 may be simply expressed as [Hav, Sav, Vav] by calculating the respective averages of H, S, and V. In another embodiment, the first scan result 1030 may be normalized. Although not exemplified, various known operations for preprocessing learning data in the field of supervised learning may be referenced to improve the learning performance of the artificial intelligence model 1010.

In connection with the learning data, in an embodiment, the learning data may further include a pair of the first label 1020 and the second scan result regarding the first model, in addition to the pair of the first label 1020 and the first scan result 1030. Here, the scan conditions (e.g., scan angle, lighting, etc.) of the second scan result may be at least partially different from the scan conditions of the first scan result. According to the present embodiment, multiple learning data may be secured by changing the scan conditions for the same first model. As a result, input color information acquired under various scan conditions may be reliably classified into one of the reference color groups.

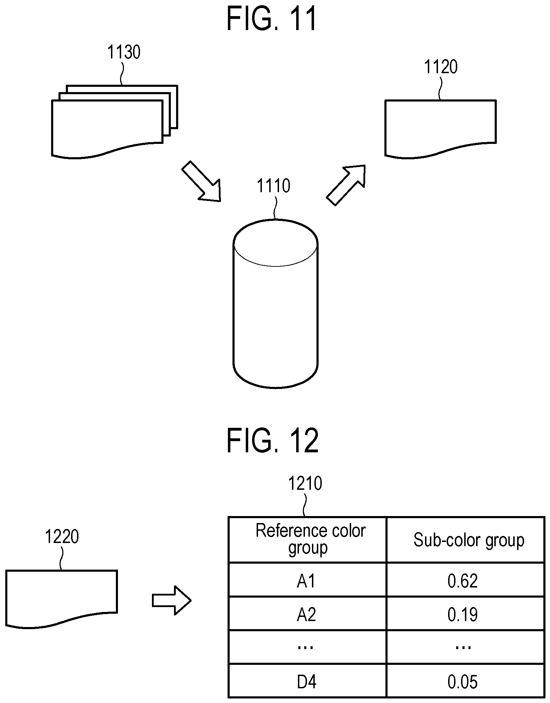

FIGS. 11 and 12 are diagrams illustrating an operation of calculating color similarity based on an artificial intelligence model, which may be referenced in various embodiments of the present disclosure.

The artificial intelligence model 1110 having learned according to the description in FIG. 10 may classify the first color information set 1130 into a label 1120 of one reference color. Since the first color information set 1130 includes one or more pieces of first color information 1220, when the first color information 1220 is input as a patch into the artificial intelligence model 1110, as shown in Table 1210, a sub-color similarity may be calculated, and when sub-color similarities for all pieces of first color information included in the first color information set 1130 are calculated, a color similarity may be calculated based on the same (e.g., by calculating the average of the sub-color similarities).

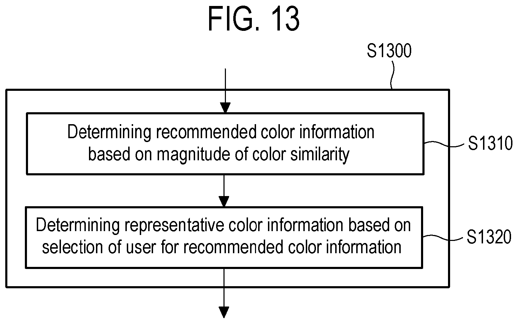

FIG. 13 illustrates a flowchart specifically illustrating the operation of determining representative color information described with reference to FIG. 6. The method S1300 illustrated in FIG. 13 may include details of the operation of determining the representative color information (S640) in FIG. 6. Hereinafter, the operations illustrated in FIG. 13 will be described.

Based on the magnitude of color similarity, recommended color information may be determined (S13 10).

The recommended color information may be a reference color capable of being recommended for the first unit object corresponding to the first point selected by the user 10. The recommended color information may be determined in the order of the magnitude of color similarity. The recommended color information may be recommended to the user 10 by being displayed on the display 107 of the electronic device 100 in a form recognizable to the user 10.

In connection with the number of pieces of recommended color information, in an embodiment, the number of pieces of recommended color information may be limited for the convenience of selection by the user 10. That is, as the number of pieces of recommended color information increases, the selection of the user 10 may become relatively difficult, so the number of pieces of recommended color information may be limited according to a predetermined rule.

For example, the upper M (where M is a natural number) reference colors may be determined as recommended color information in order of the magnitude of color similarity. Here, M may be 3, but the number of pieces of recommended color information may vary depending on the actual implementation case.

As another example, a reference color having a magnitude of color similarity exceeding a reference value may be determined as the recommended color information. In other words, a reference color whose magnitude of color similarity is less than or equal to the reference value may not be included in the recommended color information. At this time, among the reference colors whose magnitudes of color similarities exceed the reference value, up to M uppermost reference colors in the order of the magnitude of color similarity may be determined as the recommended color information (that is, if the number of reference colors exceeding the reference value is less than M, only less than M reference colors exceeding the reference value are determined as the recommended color information). Since the case where the color similarity is less than the reference value corresponds to the case where the user 10 may determine that the recommended color information is dissimilar to the actual color of the tooth, according to this operation, the reference color whose magnitude of color similarity is less than or equal to the reference value is not included in the recommended color information, thereby improving the reliability of the recommendation perceived by the user 10.

Based on the selection of the user 10 for the recommended color information, representative color information may be determined (S 1320). According to this operation, considering that even standard color information having the greatest magnitude of color similarity may not match the intention of the user 10, the user 10 may directly select one of the recommended color information, instead of automatically determining the representative color information according to the magnitude of the color similarity. Thereby, the objective information of decision on the color of the teeth and the clinical experience of the user 10 may be harmonized.

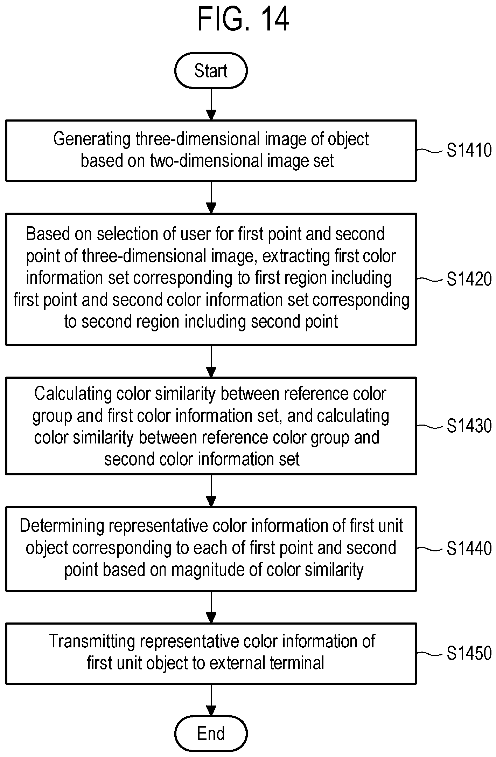

FIG. 14 illustrates a flowchart illustrating a method according to an embodiment of the present disclosure. FIG. 14 may include a series of operations triggered based on the selection of the user 10 for a plurality of points (e.g., the first point or the second point) on the three-dimensional image of the object. Hereinafter, the operations illustrated in FIG. 14 will be described.

Based on the two-dimensional image set, a three-dimensional image of the object may be generated (S1410). This operation may be understood with reference to the description of the three-dimensional image generation operation (S610) in FIG. 6.

Based on the selection of the user 10 for the first point and the second point of the three-dimensional image, a first color information set corresponding to the first region including the first point and a second color information set corresponding to the second region including the second point may be extracted (S1420). The second point is a point included in the first unit object and may indicate a point that is distinct from the first point. In addition, the second point may be treated in the same way as the first point, so the second region and the second color information set may be understood with reference to the description of the operation of extracting the first color information set in FIG. 6 (S620) regarding the first region and the first color information set, respectively.

A color similarity between the reference color group and the first color information set may be calculated, and a color similarity between the reference color group and the second color information set may be calculated (S1430). This operation is identical to the operation of using the artificial intelligence model (S630) in FIG. 6 except that the color similarity is respectively calculated for the second color information set and the first color information set, and thus may be understood with reference to the description of the operation of using the artificial intelligence model (S630) in FIG. 6.

Based on the magnitude of color similarity, representative color information of the first unit object corresponding to each of the first point and the second point may be determined (S1440). This operation is identical to the operation of determining the representative color information (S640) in FIG. 6 except that the representative color information is respectively determined for the first point and the second point, and thus may be understood with reference to the description of the operation of determining the representative color information (S640) in FIG. 6.

The representative color information of the first unit object may be transmitted to an external terminal (S1450). This operation may be understood with reference to the description of the operation of transmitting the representative color information (S650) in FIG. 6.

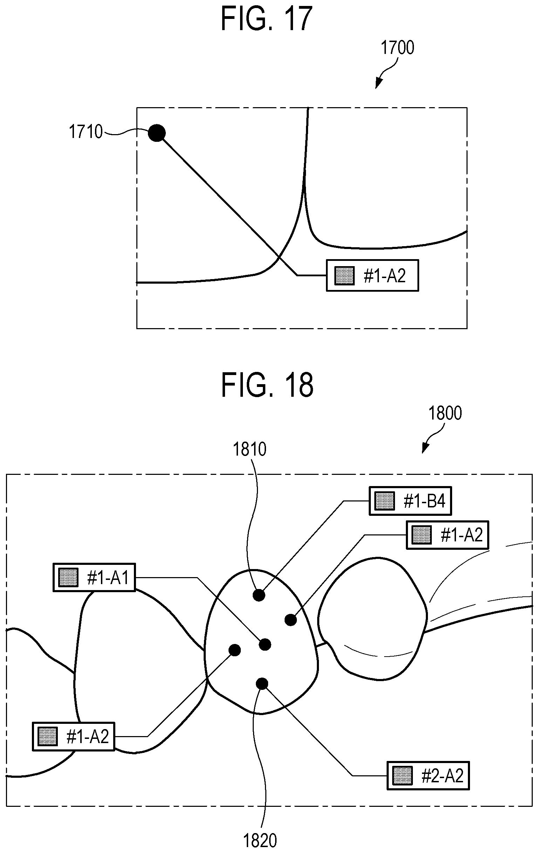

According to the embodiment illustrated in FIG. 14, by respectively processing information about the colors of a plurality of points (e.g., the first point and the second point) for one tooth, data of multiple points of the tooth may be considered in determining representative color information for the tooth (e.g., see screen 1800 in FIG. 18). Therefore, the color of the teeth of the patient 20 may be determined with greater reliability.

Although an embodiment in which the user selects the first point and the second point and then respectively determines representative color information for the first point and the second point has been described with reference to FIG. 14, the operation for each of the first point and the second point may be performed in the same manner in the case where the user selects the second point and determines representative color information for the second point after selecting only the first point and determining representative color information for the first point, or the case where the operations for the first point and the operations for the second point are performed in an arbitrary time order.

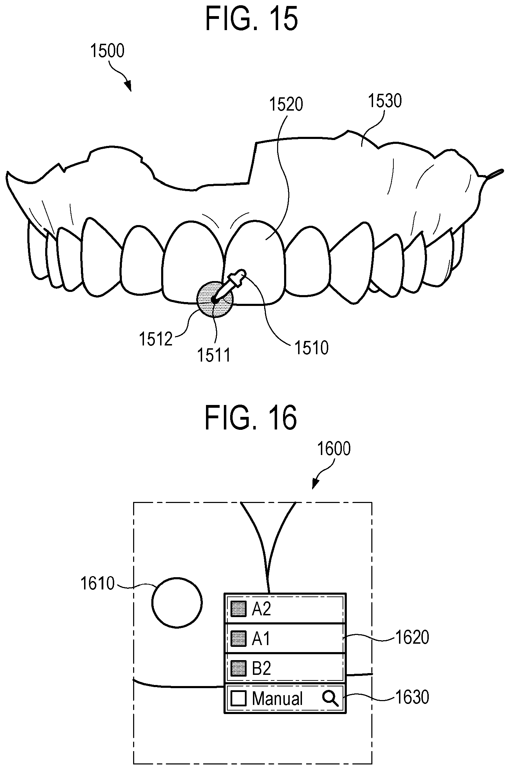

FIGS. 15 to 18 illustrate screens of a program to which a method according to an embodiment of the present disclosure is applied.

Referring to FIG. 15, a screen 1500 may include a three-dimensional image of the oral cavity (e.g., the maxilla) of a patient 20. This three-dimensional image may include a valid region, i.e., a tooth region 1520 of interest to the user 10, and a gingiva region 1530 that is an exceptional region.

In addition, the screen 1500 may include a selection tool 1510. The user 10 may select a point on the three-dimensional image by moving the selection tool 1510 through an input device 109. The selection tool 1510 may include a selection point 1511 indicating a point where the selection tool 1510 is positioned, to assist the user 10 in making a selection, and a selection region 1512 indicating a region from which information about color is extracted when the selection point 1511 is selected.

Referring to FIG. 16, a screen 1600 may include recommended color information 1620 for the tooth corresponding to the point selected by the user 10. This recommended color information 1620 may be determined from the information about the color extracted from the region 1610 corresponding to the point selected by the user 10.

In addition, the screen 1600 may further include a button 1630 that allows the user 10 to manually select the color of the tooth corresponding to the point selected by the user 10 to enhance user usability.

Referring to FIG. 17, a screen 1700 may include representative color information of the tooth corresponding to the point 1710 selected by the user 10.

The user 10 may select a plurality of points for one tooth. In this case, as shown in the screen 1800 in FIG. 18, by respectively performing the determination of the representative color information for a plurality of points 1810 and 1820, different representative color information may be determined between the points.

In an embodiment, when transmitting the representative color information to an external terminal (e.g., a dental laboratory terminal), the information about the point selected by the user 10 or the region corresponding to that point may be transmitted together. According to this embodiment, since a plurality of points may be selected for one tooth, and since the representative color information corresponding to the plurality of points may be different, even a single tooth may have different representative color information applied to different parts, and the production of prostheses may be requested according thereto.

Various embodiments of the present disclosure may be implemented as software on a machine-readable storage medium (MRSM). The software may be software for implementing various embodiments of the present disclosure. The software may be inferred from the various embodiments of the present disclosure by programmers in the art to which the present disclosure belongs. For example, the software may be a computer program containing commands readable by a computing device. The computing device may be a device capable of operating according to commands called from a storage medium and may be interchangeably referred to as, for example, an electronic device. In an embodiment, a processor of the computing device may execute the called commands, enabling the components of the computing device to perform functions corresponding to the commands. The storage medium may refer to any kind of recording medium in which information may be stored and read by a device. The storage medium may include, for example, a ROM, RAM, CD-ROM, magnetic tape, floppy disk, or optical information storage device. In an embodiment, the storage medium may be implemented in a distributed form on computer systems connected via a network. In this case, the software may be stored and executed in a distributed manner on the computer systems. In another embodiment, the storage medium may be a non-transitory storage medium. A non-transitory storage medium indicates a tangible medium that stores information semi-permanently or temporarily, excluding signals that propagate transitorily.

Although the technical spirit of the present disclosure has been described in the various embodiments, it should be noted that various substitutions, modifications, and changes may be made without departing from the scope of the present disclosure which may be understood by those skilled in the art to which the present disclosure pertains. In addition, it should be noted that that such substitutions, modifications, and changes are intended to fall within the scope of the appended claims.

## Claims

1. A method performed by an electronic device, the method comprising:
generating a three-dimensional image of an object based on a two-dimensional image set of the object obtained from an intraoral scanner, the object including a plurality of unit objects;
based on a user's selection for a first point of the three-dimensional image, extracting a first color information set corresponding to a first region including the first point;
using an artificial intelligence model configured to output a color similarity based on an input of the first color information set; and
determining representative color information of a first unit object corresponding to the first point based on a magnitude of the color similarity.

2. The method according to claim 1, further comprising transmitting the representative color information of the first unit object to an external terminal of the electronic device.

3. The method according to claim 2, wherein the transmitting representative color information to the external terminal comprises transmitting information about the first region including the first point to the external terminal.

4. The method according to claim 1, wherein the extracting the first color information set corresponding to the first region comprises determining the first region including a plurality of points within a reference distance from the first point.

5. The method according to claim 1, wherein the three-dimensional image includes a valid region and an exceptional region, and
wherein the extracting the first color information set corresponding to the first region comprises, if the first region includes the exceptional region, extracting the first color information set corresponding to the first region with the exceptional region excluded.

6. The method according to claim 1, wherein the extracting the first color information set corresponding to the first region comprises:
identifying a first two-dimensional image set corresponding to at least some of a plurality of points included in the first region, the first two-dimensional image set being a sub-set of the two-dimensional image set; and
extracting a color code of a pixel of a two-dimensional image included in the first two-dimensional image set.

7. The method according to claim 6, wherein the first two-dimensional image set includes only two-dimensional images used to generate the three-dimensional image.

8. The method according to claim 6, wherein the extracting the color code of the pixel of the two-dimensional image included in the first two-dimensional image set comprises:
determining an extraction order of two-dimensional images included in the first two-dimensional image set based on a scan order; and
extracting the color code of the pixel of the two-dimensional image included in the first two-dimensional image set based on the extraction order.

9. The method according to claim 6, wherein the first color information set includes one or more pieces of first color information having a reference size, and
wherein the extracting the first color information set corresponding to the first region further comprises generating the first color information based on the reference size.

10. The method according to claim 6, wherein the extracting the first color information set corresponding to the first region further comprises converting the extracted color code into an HSV code.

11. The method according to claim 1, wherein the first color information set includes N pieces (where N is a natural number) of first color information, and
wherein the using the artificial intelligence model comprises:
calculating sub-color similarities between respective reference colors included in a reference color group and the respective ones of the N pieces of first color information included in the first color information set; and
calculating the color similarity based on the N sub-color similarities.

12. The method according to claim 1, wherein learning data of the artificial intelligence model includes a pair of a first label indicating a first reference color included in a reference color group and a first scan result of a first model corresponding to the first reference color.

13. The method according to claim 12, wherein the learning data of the artificial intelligence model further includes a pair of the first label and a second scan result of the first model, and
wherein second scan conditions of the second scan result are at least partially different from first scan conditions of the first scan result.

14. The method according to claim 12, wherein the artificial intelligence model is a model that learns to classify, if the first scan result is input, the input of the first scan result into the first label.

15. The method according to claim 1, wherein the determining the representative color information comprises:
determining recommended color information based on the magnitude of the color similarity; and
determining the representative color information based on the user's selection for the recommended color information.

16. The method according to claim 15, wherein the determining the recommended color information comprises including a reference color whose magnitude of the color similarity exceeds a reference value in the recommended color information.

17. An electronic device comprising:
a communication circuit configured to communicate with a network;
a processor configured to execute a computer program including one or more instructions; and
a memory configured to load the computer program,
wherein the computer program comprises:
an instruction for generating a three-dimensional image of an object based on a two-dimensional image set of the object obtained from an intraoral scanner, the object including a plurality of unit objects;
an instruction for extracting, based on a user's selection for a first point of the three-dimensional image, a first color information set corresponding to a first region including the first point;
an instruction for using an artificial intelligence model configured to output a color similarity based on an input of the first color information set; and
an instruction for determining representative color information of a first unit object corresponding to the first point based on a magnitude of the color similarity.

18. A non-transitory computer-readable recording medium having a recorded computer program to be executed by a processor, the computer program comprising:
an instruction for generating a three-dimensional image of an object based on a two-dimensional image set of the object obtained from an intraoral scanner, the object including a plurality of unit objects;
an instruction for extracting, based on a user's selection for a first point of the three-dimensional image, a first color information set corresponding to a first region including the first point;
an instruction for using an artificial intelligence model configured to output a color similarity based on an input of the first color information set; and
an instruction for determining representative color information of a first unit object corresponding to the first point based on a magnitude of the color similarity.
